# EUROPÄISCHE PATENTSCHRIFT

(11) **EP 4 226 908 B1**
(45) Veröffentlichungstag und Bekanntmachung des Hinweises auf die Patenterteilung: **26.11.2025**
(21) Anmeldenummer: 23154152.5
(22) Anmeldetag: 31.01.2023
(51) Int. Cl.: A61K 8/02, A61K 8/46, A61K 8/41, A61K 8/20, A61K 8/365, A61K 8/368, A61K 8/36, A61Q 5/02, A61Q 19/10, C11D 17/06, C11D 1/14, C11D 3/04, C11D 3/30

(54) **PULVER/GRANULAT ZUR HERSTELLUNG EINES DUSCHGELS ODER SHAMPOOS**
POWDER/GRANULE FOR THE PREPARATION OF SHOWER GEL OR SHAMPOO
POUDRE/GRANULE POUR LA PRÉPARATION D'UN GEL DE DOUCHE OU D'UN SHAMPOOING

(30) Priorität: 10.02.2022 DE 102022103160
(43) Veröffentlichungstag der Anmeldung: 16.08.2023
(73) Patentinhaber: We Care² GmbH, 68163 Mannheim (DE)
(72) Erfinder: Hempel, Klaus, 69469 Weinheim (DE); Scholz, Hannah, 74936 Siegelsbach (DE); Lee, Christina, 67376 Harthausen (DE)
(74) Vertreter: Eder Schieschke & Partner mbB

(56) Entgegenhaltungen:
- EP-A2- 0 407 040
- EP-B1- 1 696 023
- WO-A1-2022/194928
- CN-A- 105 969 549
- US-A1- 2016 367 447
- US-A1- 2018 193 231
- US-A1- 2021 046 333

## Beschreibung

Die vorliegende Erfindung betrifft ein Pulver/Granulat zur Herstellung eines Duschgels oder Shampoos, das ein Salz eines Mono-C₈₋₁₈-Alkylsulfats, insbesondere ein Salz des Cocosulfats oder Laurylsulfats, und ein Ammoniumsalz aufweist, gemäß dem Patentanspruch 1. Weiterhin betrifft die vorliegende Erfindung auch die Verwendung des erfindungsgemäßen Pulvers/Granulats zur Herstellung eines Duschgels oder Shampoos gemäß Patentanspruch 6.

Herkömmliche, flüssige Shampoos und Duschgels werden normalerweise in Kunststoffverpackungen verkauft, die nach dem Verbrauch der Shampoos und Duschgels eine erhebliche Menge an Kunststoffmüll erzeugen.

Um die Menge an Kunststoffmüll zu reduzieren, kann beispielsweise ein Pulver oder Granulat bereitgestellt werden, das immer wieder in den gleichen Behälter gegeben werden kann und mit Wasser vermischt ein Shampoo oder Duschgel ergibt. Auf diese Weise kann ein nachhaltiges Produkt zur Verfügung gestellt werden, mit dem Verpackung und Transportkosten eingespart werden können.

Dabei müssen die Komponenten in Pulver- oder Granulatform vorliegen, so dass man hierbei auf Tenside angewiesen ist, die in fester Form verfügbar und gut in lauwarmem Wasser löslich sind.

Hierbei eignet sich zum Beispiel ein Salz eines Mono-C₈₋₁₈-Alkylsulfats, insbesondere ein Salz des Cocosulfats oder Laurylsulfats, da dieses als Feststoff erhältlich ist und häufig in gängigen Naturkosmetik-Duschgels und -Shampoos eingesetzt wird.

Bei der Verwendung eines Salzes eines Mono-C₈₋₁₈-Alkylsulfats, insbesondere eines Salzes des Cocosulfats oder Laurylsulfats, in Shampoos oder Duschgels wird üblicherweise ein weiteres Co-Tensid, wie zum Beispiel ein Salz des Cocoglutamats, eingesetzt, um zu verhindern, dass das Salz eines Mono-C₈₋₁₈-Alkylsulfats mit im Wasser oder in anderen Rohstoffen enthaltenen Kationen/Salzen auskristallisiert. Da fast alle bekannten Co-Tenside flüssig sind, können diese für ein Pulver oder Granulat nicht oder nur sehr begrenzt eingesetzt werden. Wird jedoch kein Co-Tensid eingesetzt, und das Salz des Mono-C₈₋₁₈-Alkylsulfats als Haupttensid verwendet, so dauert es nach dem Auflösen in warmem Leitungswasser etwa ein bis zwei Tage, bis aus dem bis dahin klaren Duschgel oder Shampoo eine weiße, cremeartige Suspension entsteht. Durch die im (harten) Leitungswasser enthaltenen Ca- und Mg-Ionen wird dieser Effekt verstärkt bzw. beschleunigt, so dass das resultierende Duschgel oder Shampoo nicht über einen Zeitraum von mehr als ein bis zwei Tagen ein klares Produkt bleibt.

Normalerweise wird ein Granulat, das anschließend in Leitungswasser aufgelöst werden soll, mit Xanthan versetzt, damit die resultierende Lösung eine gewisse Viskosität erreicht und so stabilisiert wird. Dies führt jedoch zu einem Produkt, das eine unerwünscht gelige Struktur aufweist und meist nicht klar ist. Es ist deshalb wünschenswert, ein Produkt bereitzustellen, das im Hinblick auf Durchsichtigkeit und Fließverhalten eine verbesserte Performance aufweist.

Es ist deshalb die Aufgabe der vorliegenden Erfindung, ein Pulver bzw. Granulat bereitzustellen, das vermischt mit Wasser, vorzugsweise Leitungswasser, ein klares, fließfähiges und viskoses Duschgel oder Shampoo ergibt, das über einen Zeitraum von mehreren Wochen stabil bleibt, d.h. bei dem das Tensid nicht innerhalb weniger Wochen oder Tage auskristallisiert. Dabei ist es wünschenswert, kein weiteres Co-Tensid beimischen zu müssen, da die meisten Co-Tenside in flüssiger bzw. gelöster Form vorliegen, so dass kein trockenes Pulver bzw. Granulat bereitgestellt werden kann.

Die genannte Aufgabe wird durch ein Pulver bzw. Granulat nach dem Patentanspruch 1 gelöst. Das erfindungsgemäße Pulver/Granulat dient zur Herstellung eines Duschgels oder Shampoos und weist ein Salz eines Mono-C₈₋₁₈-Alkylsulfats, insbesondere ein Salz des Cocosulfats oder Laurylsulfats, und ein Ammoniumsalz auf. Überraschenderweise haben die Erfinder der vorliegenden Erfindung festgestellt, dass durch Beimischen eines Ammoniumsalzes zum Salz eines Mono-C₈₋₁₈-Alkylsulfats keine weitere Zugabe eines Co-Tensids notwendig ist, um nach dem Auflösen des Pulvers bzw. Granulat über viele Wochen ein klares Produkt zu erhalten. Ein weiterer Vorteil der Verwendung des Ammoniumsalzes liegt darin, dass es verdickende Eigenschaften hat, so dass die für Duschgels und Shampoos übliche Konsistenz erhalten wird.

Als Salz eines Mono-C₈₋₁₈-Alkylsulfats wird in dem erfindungsgemäßen Pulver bzw. Granulat vorzugsweise ein Alkalimetall-Mono-C₈₋₁₈-Alkylsulfat oder Ammonium-Mono-C₈₋₁₈-Alkylsulfat verwendet, wobei Ammonium-, Natrium- oder Kalium-Mono-C₈₋₁₈-Alkylsulfat bevorzugt sind. Die Verwendung der zuletzt genannten Salze hat gegenüber anderen Alkalimetallen den Vorteil, dass diese günstig und leicht wasserlöslich sind, wobei das Natrium-Mono-C₈₋₁₈-Alkylsulfat stärker bevorzugt ist. Das Salz eines Mono-C₈₋₁₈-Alkylsulfats kann erfindungsgemäß auch eine Mischung verschiedener Salze eines oder mehrerer verschiedener C₈₋₁₈-Alkylsulfate, d.h. verschiedener C₈₋₁₈-Alkylsulfate mit unterschiedlichen C-Kettenlängen, wie es z.B. bei Cocosulfat der Fall ist.

Das Mono-C₈₋₁₈-Alkylsulfat ist bevorzugt ein Mono-C₁₂₋₁₈-Alkylsulfat, insbesondere Laurylsulfat oder Cocosulfat.

Besonders bevorzugt ist das Salz eines Mono-C₈₋₁₈-Alkylsulfats Natriumlaurylsulfat oder Natriumcocosulfat. Diese haben den Vorteil, dass sie besonders gut wasserlöslich sind.

Unter einem Salz eines Cocosulfats versteht man hierin ein Salz eines Mono-C₁₂₋₁₈-Alkylsulfats, bzw. eine Mischung aus verschiedenen dieser Alkylsulfate. Das Salz des Cocosulfats wird üblicherweise durch Reaktion von Kokosöl oder Palmöl mit Schwefelsäure und anschließender Reaktion mit einem Salz gewonnen.

In dem erfindungsgemäßen Pulver bzw. Granulat kann das Ammoniumsalz ein Ammoniumhalogenid, Ammoniumsulfat oder Ammoniumcarbonat sein, wobei ein Ammoniumhalogenid bevorzugt ist. Erfindungsgemäß sind als Ammoniumhalogenide Ammoniumchlorid oder -bromid bevorzugt, wobei Ammoniumchlorid stärker bevorzugt ist.

Das Ammoniumion des Ammoniumsalzes kann eine organische Ammoniumverbindung, insbesondere ein Mono-, Di- oder Tri-alkyliertes Ammoniumion, insbesondere Triethylammonium, sein. Bevorzugt ist das Ammoniumion jedoch ein NH₄⁺-Ion. Dadurch kann eine besonders gute Löslichkeit gewährleistet werden.

In einer weiteren erfindungsgemäßen Ausgestaltung kann das Pulver bzw. Granulat zusätzlich eine organische Carbonsäure aufweisen. Hierbei ist es bevorzugt, dass die organische Carbonsäure eine Mehrzahl von Carbonsäuregruppen aufweist. Besonders bevorzugt weist die organische Carbonsäure zwei, drei oder vier Carbonsäuregruppen auf, wobei eine organische Carbonsäure mit drei Carbonsäuregruppen bevorzugt ist. Vorzugsweise kann die organische Carbonsäure auch eine oder mehrere Hydroxylgruppen aufweisen. Beispiele verwendbarer organischer Carbonsäuren sind Zitronensäure oder Milchsäure. Die organische Carbonsäure liegt vorzugsweise in einer Menge von 2,5 Gew.-% bis 15 Gew.-% in dem Pulver bzw. Granulat vor, bezogen auf das Gesamtgewicht des Pulvers/Granulats. Der Einsatz von organischen Säuren ermöglicht die Einstellung des pH-Werts, um das resultierende Duschgel oder Shampoo in einem pH-Wertbereich von weniger als 5, vorzugsweise aber nicht weniger als 2 einzustellen. Dies hat den Vorteil, dass bei Einsatz eines Konservierungsmittels, wie beispielsweise Benzoesäure, dieses in einer für die Konservierung ausreichender Form als freie Säure vorliegt.

In einer weiteren erfindungsgemäßen Ausgestaltung weist das Pulver/Granulat vorzugsweise 50 Gew.-% bis 80 Gew.-%. und am stärksten bevorzugt 60 Gew.-% bis 70 Gew.-% des Salzes eines Mono-C₈₋₁₈-Alkylsulfats, insbesondere des Salzes des Cocosulfats oder Laurylsulfats, auf, jeweils bezogen auf das Gesamtgewicht des Pulvers/Granulats. Bei einer zu hohen Menge löst sich das Salz eines Mono-C₈₋₁₈-Alkylsulfats, insbesondere das Salz des Cocosulfats oder Laurylsulfats, nicht ausreichend in Wasser. Bei einer zu geringen Menge hat das daraus resultierende Duschgel bzw. Shampoo keine ausreichenden Wascheigenschaften.

In einer weiteren erfindungsgemäßen Ausgestaltung weist das Pulver/Granulat vorzugsweise bis 30 Gew.-% und am stärksten bevorzugt 15 Gew.-% bis 25 Gew.-% des Ammoniumsalzes auf, jeweils bezogen auf das Gesamtgewicht des Pulvers/Granulats. Bei einer zu hohen Menge ist das resultierende Duschgel bzw. Shampoo zu viskos. Bei einer zu geringen Menge kristallisiert das Salz eines Mono-C₈₋₁₈-Alkylsulfats, insbesondere das Salz des Cocosulfats oder Laurylsulfats, nach wenigen Tagen aus.

Gemäß einer weiteren bevorzugten erfindungsgemäßen Ausgestaltung weist das Pulver/Granulat einen Stoffmengenüberschuss von Ammoniumionen zu Mono-C₈₋₁₈-Alkylsulfat-Ionen, insbesondere Cocosulfationen oder Laurylsulfationen, auf, insbesondere einen Stoffmengenüberschuss im Bereich von 20 zu 1 bis 1,1 zu 1, stärker bevorzugt 10 zu 1 bis 1,3 zu 1, und noch stärker bevorzugt 5 zu 1 bis 1,5 zu 1. Ein solcher Überschuss sorgt für eine gute Löslichkeit des Pulvers/Granulats und verhindert eine Trübung des daraus hergestellten Duschgels/Shampoos. Bei einer zu niedrigen Menge an Ammoniumionen kann keine klare Mischung erhalten werden.

Weist das erfindungsgemäße Pulver/Granulat ein weiteres Co-Tensid auf, so liegt dieses maximal in einer Gewichtsmenge von weniger als der Hälfte der Gewichtsmenge des des Salzes eines Mono-C₈₋₁₈-Alkylsulfats, insbesondere des Salzes des Cocosulfats oder Laurylsulfats, vor, bezogen auf das Gesamtgewicht des Pulvers/Granulats. Der diesbezügliche Vorteil, nämlich maximal geringe Mengen an weiteren Co-Tensiden einzusetzen, liegt darin, dass durch die Verwendung des Ammoniumsalzes eine Ausfällung des Salzes des Salzes eines Mono-C₈₋₁₈-Alkylsulfats, insbesondere des Salzes des Cocosulfats oder Laurylsulfats, verhindert werden kann. Besonders bevorzugt ist es erfindungsgemäß, dass das erfindungsgemäße Pulver/Granulat kein weiteres (flüssiges) Co-Tensid aufweist.

Neben den bisher genannten Bestandteilen kann das erfindungsgemäße Pulver/Granulat auch noch weitere Bestandteile wie z.B. Konservierungsstoffe oder Duftstoffe aufweisen. Beispiele für Konservierungsstoffe sind Natriumbenzoat und Kaliumsorbat. Ein Beispiel für einen Duftstoff ist das Parfümöl Blue Care 9070211. Es ist erfindungsgemäß bevorzugt, dass Konservierungsstoffe in einer Menge von 0 Gew.-% bis 8 Gew.-%, stärker bevorzugt in einer Menge von 0 Gew.-% bis 5 Gew.-% eingesetzt werden, bezogen auf das Gesamtgewicht des Pulvers/Granulats. Weiterhin ist es bevorzugt, dass der Duftstoff in einer Menge von 0 Gew.-% bis 8 Gew.-%, stärker bevorzugt in einer Menge von 0 Gew.-% bis 5 Gew.-% eingesetzt wird, bezogen auf das Gesamtgewicht des Pulvers/Granulats.

Die vorliegende Erfindung betrifft auch ein Pulver/Granulat, das aus den folgenden Bestandteilen besteht:
- 40 Gew.-% bis 80 Gew.-%, stärker bevorzugt 50 Gew.-% bis 80 Gew.-%, und am stärksten bevorzugt 60 Gew.-% bis 70 Gew.-% eines Salzes eines Mono-C₈₋₁₈-Alkylsulfats, insbesondere eines Salzes des Cocosulfats oder Laurylsulfats;
- 15 Gew.-% bis 40 Gew.-%, stärker bevorzugt bis 30 Gew.-% und am stärksten bevorzugt 15 Gew.-% bis 25 Gew.-% eines Ammoniumsalzes;
- 0 Gew.-% bis 15 Gew.-%, stärker bevorzugt 2,5 Gew.-% bis 15 Gew.-% organische Carbonsäure;
- 0 Gew.-% bis 8 Gew.-%, stärker bevorzugt 0 Gew.-% bis 5 Gew.-% Konservierungsstoff; und
- 0 Gew.-% bis 8 Gew.-%, stärker bevorzugt 0 Gew.-% bis 5 Gew.-% Duftstoff, jeweils bezogen auf das Gesamtgewicht des Pulvers/Granulats.

Eine weitere Ausführungsform der Erfindung betrifft die Verwendung eines erfindungsgemäßen Pulvers/Granulats zur Herstellung eines Duschgels oder Shampoos. Hierfür wird das erfindungsgemäße Pulver/Granulat mit Wasser, vorzugsweise Leitungswasser, vermischt. In anderen Worten betrifft die vorliegende Erfindung auch ein Verfahren zur Herstellung eines Duschgels oder Shampoos, in dem das erfindungsgemäße Pulver/Granulat mit Wasser vermischt wird. Dabei ist es bevorzugt, dass Wasser in einer Menge zu dem Pulver/Granulat zugegeben wird, dass in dem resultierenden Duschgel oder Shampoo das Gewichtsverhältnis von Pulver/Granulat zu Wasser im Bereich von 1:4 bis 1:20, stärker bevorzugt im Bereich von 1:6 bis 1:15 und am stärksten bevorzugt im Bereich 1:8 bis 1:10 liegt. Eine zu hohe Menge an Wasser führt zu einem zu wässrigen Produkt mit geringerer Waschleistung. Bei einer zu geringen Menge an Wasser ist das Produkt zu viskos für den Anwendungsbereich, oder das Pulver/Granulat löst sich nicht vollständig.

Hiermit wird auch beschrieben ein Duschgel/Shampoo, das ein Salz eines Mono-C₈₋₁₈-Alkylsulfats, insbesondere ein Salz des Cocosulfats oder Laurylsulfats, ein Ammoniumsalz und Wasser aufweist, das dadurch gekennzeichnet ist, dass ein weiteres Co-Tensid maximal in einer Gewichtsmenge von weniger als der Hälfte der Gewichtsmenge des Salzes eines Mono-C₈₋₁₈-Alkylsulfats, insbesondere eines Salzes des Cocosulfats oder Laurylsulfats, vorliegt. Solch geringe Mengen an einem weiteren Co-Tensid können deshalb eingesetzt werden, da die Kombination aus Salz eines Mono-C₈₋₁₈-Alkylsulfats, insbesondere Salz des Cocosulfats oder Laurylsulfats, mit einem Ammoniumsalz bereits das Ausfällen des Salzes eines Mono-C₈₋₁₈-Alkylsulfats, insbesondere eines Salzes des Cocosulfats oder Laurylsulfats, über mehrere Wochen verhindert. Deshalb ist es erfindungsgemäß auch möglich, auf diese Weise ein klares, über Wochen stabiles Duschgel/Shampoo bereitzustellen, das im Wesentlichen kein weiteres Co-Tensid aufweist.

Als Wasser zur Herstellung des erfindungsgemäßen Duschgels oder Shampoos kann demineralisiertes oder mineralhaltiges Wasser verwendet werden. Insbesondere soll das erfindungsgemäße Pulver/Granulat jedoch in normalem Leitungswasser gut löslich und über einen Zeitraum von mehreren Wochen stabil sein.

Besonders bevorzugt ist das erfindungsgemäße Duschgel/Shampoo eines, das dadurch erhältlich ist, dass ein erfindungsgemäßes Pulver/Granulat mit Wasser vermischt wird.

Gemäß einer weiteren bevorzugten erfindungsgemäßen Ausgestaltung weist das Duschgel/Shampoo vorzugsweise eine Viskosität im Bereich von 2.000 mPa*s bis 15.000 mPa*s, und stärker bevorzugt im Bereich von 3.000 mPa*s bis 7000 mPa*s auf, die nach Brookfield bei 20°C (Spindel 05, 20 rpm) gemessen wird. Bei einer solchen Viskosität lässt sich das Duschgel/Shampoo besonders gut handhaben und weist ein angenehmes Empfinden bei der Verwendung auf.

Gemäß einer weiteren bevorzugten erfindungsgemäßen Ausgestaltung weist das Duschgel/Shampoo vorzugsweise eine Trübung von weniger als 20 NFU, stärker bevorzugt weniger 10 NFU und noch stärker bevorzugt von weniger als 5 NFU auf, gemessen nach DIN EN ISO 7027-1. Ein solches Duschgel ist besonders klar und durchsichtig und erweckt einen gewünschten optischen Eindruck.

Bevorzugt kann das erfindungsgemäße Duschgel/Shampoo bei einer Bestimmung der Fließfähigkeit bei 20 °C in einem Kegel-Platte-Messsystem, eine Fließgrenze von mindestens 50 Pa, bestimmt bei einer Variation der Schubspannung von 0,4 bis 100 Pa im Schubspannungsversuch, aufweisen und/oder eine Fließgrenze von mindestens 65 Pa, bestimmt bei einer Variation der Scherrate von 0,1 bis 1000 s⁻¹ im Schergeschwindigkeitsversuch, aufweisen. Die Erstellung der Messkurve kann via Hershell-Bulkley-Regression erfolgen. Beispielsweise kann ein Kegel-Platte-Messsystem RCT-50-1 Brookfield verwendet werden. Diese Parameter spiegeln das haptische Verhalten, bzw. die Fließfähigkeit, sowie die Verteilbarkeit des Duschgels/Shampoos wider. Eine derartige Fließfähigkeit und Verteilbarkeit ist besonders vorteilhaft.

Weiter kann das erfindungsgemäße Duschgel/Shampoo bei einer Thixotropie-Messung im Bereich von 0,4 bis 50 Pa eine Thixotropie-Fläche von unter 10000 Pa*s, insbesondere von unter 1000 Pa*s, aufweisen. Eine solche geringe Thixotropie gewährleistet gleichmäßige Fließeigenschaften und damit eine gleichmäßige Verteilbarkeit des Duschgels/Shampoos.

Die vorliegende Erfindung soll nun anhand folgender Beispiele erläutert werden:

### Beispiel 1: Herstellung eines erfindungsgemäßen Granulats bzw. Duschgels/Shampoos

Gemäß der folgenden Tabelle 1 wurde ein erfindungsgemäßes Granulat mit den darin angegeben Bestandteilen hergestellt. Weiterhin wurden 12 g des Granulats mit 88 g Leitungswasser (25°C) zu einem Shampoo/Duschgel vermischt. Nach dem vollständigen Auflösen ergab sich eine klare Mischung, die in einem Temperaturfenster im Bereich von 5°C bis 40°C über 3 Monate unverändert blieb. Bei Temperaturen um 5°C kam es zwischenzeitlich zur Bildung von Kristallen, die sich jedoch leicht bei einer Erhöhung der Temperatur um 10°C bis 15°C wieder auflösen ließen.

**Tabelle 1:**

| **Bestandteil** | **Name** | **Name** | **Granulat (Anteile der Bestandteile)** | **Shampoo/ Duschgel (Anteile der Bestandte ile)** |
|---|---|---|---|---|
| | | | [Gew.-%] | [Gew.-%] |
| Lösungsmittel | WASSER DEMIN. | | | 88 |
| Tensid anionisch | SODIUM COCO-SULFATE MB | Sulfopon 1216G; BASF | 65,84 | 7,90 |
| Hilfsstoff | AMMONIUMCHLORID | Chemsolte 2668; TH Geyer | 20,83 | 2,50 |
| Organische Carbonsäure | ZITRONENSÄURE DAB (GRIES) | Zitronensäure DAB (Gries); Brenntag GmbH | 7,5 | 0,90 |
| Konservierungsstoff | NATRIUMBENZOAT | Natriumbenzoat Prills 25; RFI Food Ingredients | 1,67 | 0,20 |
| Konservierungsstoff | KALIUMSORBAT | Kaliumsorbat ZTN; Ter Hell | 0,83 | 0,10 |
| Parfümöl | PÖ BLUE CARE 9070211 | Parfüm Blue Care; Robertet S.A. | 3,33 | 0,40 |

### Vergleichsbeispiele 1 bis 4 mit Alkalichloriden anstelle von Ammoniumchlorid:

Anstelle der in Tabelle 1 angegebenen Menge an Ammoniumchlorid wurden hier jeweils NaCl (Vergleichsbeispiel 1), KCI (Vergleichsbeispiel 2), CaCl₂ (Vergleichsbeispiel 3) und MgCl₂ (Vergleichsbeispiel 4) eingesetzt. Nach dem Auflösen in demineralisiertem Wasser analog zum erfindungsgemäßen Beispiel 1 konnten im Falle des NaCl, KCI und MgCl₂ zunächst jeweils klare Duschgele/Shampoos erhalten werden, bei denen jedoch nach ein bis zwei Tagen eine Trübung durch Ausfallen des Natriumcocosulfats beobachtet wurde. Im Falle des CaCl₂ konnte durch Zugabe von Wasser von Anfang an kein klares Produkt erhalten werden.

### Vergleichsbeispiele 5 und 6 mit anderen Tensiden anstelle von Natriumcocosulfat:

In weiteren Vergleichsversuchen wurden anstelle der in Tabelle 1 angegebenen Menge an Natriumcocosulfat jeweils Natriumcocoylisethionat (Vergleichsbeispiel 5) bzw. Natriumlaurylsulfoacetat (Vergleichsbeispiel 6) eingesetzt. Das erhaltene Granulat ließ sich in beiden Fällen sehr schlecht in Wasser lösen und ergab kein klares Produkt.

### Vergleichsbeispiel 7:

Beim Einsatz von Ammoniumcocosulfat ohne ein weiteres Ammoniumsalz ließ sich das erhaltene Granulat ebenfalls sehr schlecht in Wasser lösen und ergab kein klares Produkt.

### Vergleichsbeispiel 8: Pulver/Granulat mit Xanthan als Hilfsstoff/Stabilisator anstelle von Ammoniumsalz:

Hier wurde ein Pulver/Granulat mit folgenden Inhaltsstoffen bereitgestellt:
Natriumcocosulfat, Xanthan, Theobroma cacao seed butter, Zitronensäure, Kaliumsorbat, Kieselsäuregel, Natriumbenzoat, Butyrospermum parkii butter, Aloe barbadensis leaf juice, Camellia sinensis leaf powder, CI 75100, Gardenia jasminoides fruit extract, Maltodextrin, Limonen, Benzylsalicylat, Eugenol, Parfum

### Vergleichsbeispiel 9: Standardshampoo:

Das Standardshampoo enthält folgende Zusammensetzung:

**Tabelle 2: Angaben in Gewichtsprozent**

| | |
|---|---|
| WASSER DEMIN. | 78.0899475 |
| ETHERSULFAT MB | 10.8 |
| Cocamidopropyl Betain | 5.0 |
| GLYCERIN 99,5 % | 2.0 |
| TOTES MEER SALZ | 1.5 |
| LAMESOFT PO 65 MB | 1.0 |
| WEISSER TEE EXTR A KBA 0485621 | 0.1 |
| OLIVENBLÄTTER EXTR GLY 0487304 | 0.01 |
| PÖ WHITE TEA & MAGNOLIA 68404 | 0.5 |
| NATRIUMBENZOAT | 0.4 |
| KALIUMSORBAT | 0.2 |
| ZITRONENSÄURE DAB (GRIES) | 0.4 |
| SPEISESALZ GROB (SOLE) | 0.0000025 |
| FD & C BLUE NO.1, C.I.42090 100301 | 0.00005 |

Zur Herstellung eines Shampoos/Duschgels werden 20 g des Pulvers zügig in 240 ml lauwarmes Wasser gegeben und sofort 10-mal kräftig geschüttelt. Dann lässt man für 15 Minuten stehen und schüttelt noch einmal kräftig.

### Untersuchungen verschiedener Eigenschaften der Zusammensetzungen des Beispiels 1 und der Vergleichsbeispiele 8 und 9:

### 1. Schaumverhalten:

Aus den Shampoos/Duschgels des Beispiels 1 und der Vergleichsbeispiele 8 und 9 werden jeweils 5 Gew.-%ige wässrige Tensidlösungen hergestellt. Von den Tensidlösungen werden jeweils 1 g in einen 10 ml Standzylinder gefüllt. Jede der Tensidlösungen wird für 10 s gleichmäßig geschüttelt.

Das erfindungsgemäße Shampoo/Duschgel des Beispiels 1 schäumt hierbei bis zur 5 ml Markierung des Standzylinders, wohingegen die Tensidlösung nach Vergleichsbeispiel 8 nur bis zur 2 ml Markierung und die Tensidlösung nach Vergleichsbeispiel 9 nur bis zu 5 ml Markierung schäumt.

In anderen Worten schneidet das erfindungsgemäße Shampoo nach Beispiel 1 bzgl. des Schaumverhaltens besser ab als das Shampoo nach Vergleichsbeispiel 8 und deutlich besser ab als das Standardshampoo nach Vergleichsbeispiel 9.

Weiterhin wird die Schaumstabilität nach 20 Minuten und 60 Minuten Stehenlassen untersucht:
Bei allen Shampoos ist die Schaumstabilität vergleichbar gut, d.h. die Schaummenge (Füllstand des Standzylinders) nimmt in diesem Zeitraum kaum merklich ab.

### 2. Trübung

Für die nach Beispiel 1 und Vergleichsbeispiel 8 hergestellten Shampoos wird die Trübung nach DIN EN ISO 7027-1 bestimmt. Dabei erhält man für das Shampoo nach Beispiel 1 einen Wert von 4,6 FNU und für das Shampoo nach Vergleichsbeispiel 8 einen Wert von >1000 FNU, d.h. einen Wert, der oberhalb des Messbereichs liegt.

Daraus ergibt sich für das erfindungsgemäße Shampoo, dass dieses im Vergleich zu Shampoos mit Xanthan als Verdicker eine deutlich geringere Trübung aufweist, die vom Verbraucher als angenehm und wünschenswert wahrgenommen wird.

### 3. Fließfähigkeit und haptisches verhalten

Zur Bestimmung der Fließfähigkeit und des haptischen Verhaltens der Shampoos aus Beispiel 1 und Vergleichsbeispiel 8 wird über die Variation der Schubspannung und der Scherrate die Thixothropie in einem Viskositätsdiagramm bestimmt, die ein Maß für die Fließfähigkeit und das haptische Verhalten der resultierenden Shampoos ist. Je geringer die Thixotropie, desto besser sind die Fließfähigkeit bzw. das haptische Verhalten des Shampoos.

Hierfür werden folgende Geräte verwendet:
- Rheometer RSTCPS, Brookfield
- Thermostat RST-TC-PA, Brookfield
- Kegel-Platte-Messsystem RCT-50-1, Brookfield
- Messsoftware Rheo 3000 Version 2.1.109.24663, Brookfield

Hierfür wird in den jeweiligen Messversuchen die Schubspannung gegen die Scherrate aufgetragen. Dabei werden 300 Messpunkte bei einer konstanten Temperatur von 20°C aufgenommen. Die Durchführung erfolgt in Doppelbestimmung. Die Auswertung erfolgt visuell. Die Messkurve wird via Hershel-Bulkley-Regression erstellt.

Messeinstellungen:
- Variation der Schubspannung: 0,4 bis 100 Pa
- Variation der Scherrate: 0,1 bis 1000 1/s
- Thixotropie-Messungen: 0,4 bis 50 Pa

Die Messergebnisse ergaben für das erfindungsgemäße Shampoo des Beispiels 1 eine Thixotropie-Fläche von 513 Pa*s und für das Shampoo des Vergleichsbeispiels 8 eine Thixotropie-Fläche von 15639 Pa*s.

Daraus ergibt sich für das erfindungsgemäße Shampoo ein nur geringfügiges thixotropes Verhalten und somit eine hervorragende Fließrate und haptisches Verhalten. Das Vergleichsshampoo zeigt hingegen ein deutlich thixotropes Verhalten und somit wesentlich geringere Fließeigenschaften und haptisches Verhalten.

## Patentansprüche

1. Pulver/Granulat zur Herstellung eines Duschgels oder Shampoos, das 40 Gew.-% bis 80 Gew.-% eines Salzes eines Mono-C₈₋₁₈-Alkylsulfats, insbesondere eines Salzes des Cocosulfats oder Laurylsulfats, und 15 Gew.-% bis 40 Gew.-% eines Ammoniumsalzes aufweist, jeweils bezogen auf das Gesamtgewicht des Pulvers/Granulats.

2. Pulver/Granulat nach Anspruch 1, worin das Salz des Mono-C₈₋₁₈-Alkylsulfats Ammonium-Mono-C₈₋₁₈-Alkylsulfat oder ein Alkali-Mono-C₈₋₁₈-Alkylsulfat ist.

3. Pulver/Granulat nach Anspruch 1 oder 2, worin das Mono-C₈₋₁₈-Alkylsulfat ein Mono-C₁₂₋₁₈-Alkylsulfat, insbesondere Cocosulfat oder Laurylsulfat ist.

4. Pulver/Granulat nach einem der Ansprüche 1 bis 3, worin das Ammoniumsalz ein Ammoniumhalogenid ist.

5. Pulver/Granulat nach einem der Ansprüche 1 bis 4, das einen Stoffmengenüberschuss von Ammoniumionen zu Mono-C₈₋₁₈-Alkylsulfat-Ionen aufweist, insbesondere einen Stoffmengenüberschuss im Bereich von 20 zu 1 bis 1,1 zu 1, stärker bevorzugt 10 zu 1 bis 1,3 zu 1, noch stärker bevorzugt 5 zu 1 bis 1,5 zu 1

6. Verwendung eines Pulvers/Granulats gemäß einem der Ansprüche 1 bis 5 zur Herstellung eines Duschgels oder Shampoos.

7. Verwendung nach Anspruch 6, worin das Pulver/Granulat mit Wasser vermischt wird.

## Claims

1. Powder/granular material for producing a shower gel or shampoo, which contains 40 wt.-% to 80 wt.-% of a salt of a mono-C₈₋₁₈-alkyl sulfate, in particular a salt of coco sulfate or lauryl sulfate, and 15 wt.-% to 40 wt.-% of an ammonium salt, each relative to the total weight of the powder/granular material.

2. Powder/granular material according to claim 1, in which the salt of the mono-C₈₋₁₈-alkyl sulfate is ammonium mono-C₈₋₁₈-alkyl sulfate or an alkali mono-C₈₋₁₈-alkyl sulfate.

3. Powder/granular material according to claim 1 or 2, in which the mono-C₈₋₁₈-alkyl sulfate is a mono-C₁₂₋₁₈-alkyl sulfate, in particular coco sulfate or lauryl sulfate.

4. Powder/granular material according to one of claims 1 to 3, in which the ammonium salt is an ammonium halide.

5. Powder/granular material according to one of claims 1 to 4, which contains a molar excess of ammonium ions relative to mono-C₈₋₁₈-alkyl sulfate ions, in particular a molar excess in the range of from 20 to 1 to 1.1 to 1, more preferably 10 to 1 to 1.3 to 1, even more preferably 5 to 1 to 1.5 to 1.

6. Use of a powder/granular material according to one of claims 1 to 5 for producing a shower gel or shampoo.

7. Use according to claim 6, in which the powder/granular material is mixed with water.

## Revendications

1. Poudre/granulés pour la fabrication d'un gel douche ou d'un shampooing, lesquels contiennent 40 % en poids à 80 % en poids d'un sel d'un monoalkylsulfate en C₈₋₁₈, en particulier d'un sel de coco sulfate ou de lauryl sulfate, et 15 % en poids à 40 % en poids d'un sel d'ammonium, respectivement par rapport au poids total de la poudre/des granulés.

2. Poudre/granulés selon la revendication 1, dans lesquels le sel du monoalkylsulfate en C₈₋₁₈ est un monoalkylsulfate en C₈₋₁₈ d'ammonium ou un monoalkylsulfate en C₈₋₁₈ alcalin.

3. Poudre/granulés selon la revendication 1 ou 2, dans lesquels le monoalkylsulfate en C₈₋₁₈ est un monoalkylsulfate en C₁₂₋₁₈, en particulier le coco sulfate ou le lauryl sulfate.

4. Poudre/granulés selon l'une des revendications 1 à 3, dans lesquels le sel d'ammonium est un halogénure d'ammonium.

5. Poudre/granulés selon l'une des revendications 1 à 4, lesquels présentent un excès de quantité de matière d'ions ammonium par rapport aux ions monoalkylsulfate en C₈₋₁₈, en particulier un excès de quantité de matière dans la plage allant de 20 à 1 à 1,1 à 1, plus préférablement de 10 à 1 à 1,3 à 1, encore plus préférablement de 5 à 1 à 1,5 à 1.

6. Utilisation d'une poudre/de granulés selon l'une des revendications 1 à 5 pour la fabrication d'un gel douche ou d'un shampooing.

7. Utilisation selon la revendication 6, dans laquelle la poudre/les granulés sont mélangés à de l'eau.
